# EUROPÄISCHE PATENTANMELDUNG

(11) **EP 1 400 908 A1**
(43) Veröffentlichungstag der Anmeldung: **24.03.2004**
(21) Anmeldenummer: 02021128.0
(22) Anmeldetag: 23.09.2002
(51) Int. Cl.: G06F 19/00

(54) **Verfahren und Vorrichtung zur Überprüfung von Kodierungen im Gesundheitswesen**

(71) Anmelder: SIEMENS AKTIENGESELLSCHAFT, 80333 München (DE)
(72) Erfinder: Mankopf, Michael, 91096 Möhrendorf (DE); Schmidt, Volker, Dr., 91054 Erlangen (DE)

(57) **Zusammenfassung**

Die Erfindung betrifft ein Verfahren für eine Überprüfung von einem Patienten (3, 31) zugeordneten Kodierungen im Gesundheitswesen, wobei die Kodierungen einem medizinischen Profil des Patienten (3, 31) zugeordnet sind. Die Erfindung betrifft außerdem eine Vorrichtung für eine Überprüfung von einem Patienten (3, 31) zugeordneten Kodierungen im Gesundheitswesen.

## Beschreibung

Die Erfindung betrifft ein Verfahren und eine Vorrichtung zur Überprüfung von einem Patienten zugeordneten Kodierungen im Gesundheitswesen.

Im Gesundheitswesen ist es üblich, medizinische Sachverhalte, wie beispielsweise eine Diagnose für einen Patienten oder an dem Patienten durchgeführte medizinische Prozeduren, wie z.B. Operationen oder Untersuchungen, mittels spezieller international geläufiger Kodierungssysteme zu kodieren. In Deutschland werden beispielsweise medizinische Prozeduren nach dem International Classification of Procedures in Medicine (ICPM) bzw. der internationalen Klassifizierung der Prozeduren in der Medizin (IKPM) gemäß § 301 SGB V kodiert.

Solche Kodierungen dienen u.a. als Grundlage für die Vergütung der Ärzte bzw. Krankenhäuser. So wird beispielsweise das sogenannte DRG-Kodierungssystem (Diagnosis Related Groups) im US-Bundesstaat New Jersey zur Vergütung stationär behandelter medicare Versicherter eingesetzt.

Gemäß einer Pressemitteilung vom 1. Dezember 2000 des Deutschen Instituts für medizinische Dokumentation und Information (DIMDI) stehen die International Classification of Diseases-10 (ICD-10) und der Operationenschlüssel nach § 301 SGB V in der Version 2.0 bereit. Beide Klassifikationen wurden für die Einführung eines pauschalisierten Entgeltsystems auf der Grundlage der Australien Refined Diagnosis Related Groups (AR-DRGs) umfassend revidiert.

Die oben stehenden Kodierungssysteme sind kompliziert; bisweilen werden deshalb medizinische Sachverhalte versehentlich falsch kodiert. Da die Kodierungen auch Grundlage der Vergütung sind, werden medizinische Sachverhalte auch manchmal in betrügerischer Absicht falsch kodiert.

In der DE 198 25 191 A1 ist eine Vorrichtung zum Erfassen, Verarbeiten und Protokollieren von patientenbezogenen Daten im Vorfelde und während einer medizinischen Behandlung offenbart. Die Vorrichtung umfasst einen Server mit Zugriff auf eine Datenbank, in der patientenbezogenen Daten gespeichert sind, und wenigstens einen Client, der mit dem Server vernetzt ist. Der Client kann auf die patientenbezogenen Daten der Datenbank und der Server auf vom Client abrufbare Daten zugreifen. Die Vorrichtung umfasst außerdem wenigstens ein mobiles Datenerfassungssystem und Mittel zur Datenübertragung zwischen dem mobilen Datenerfassungssystem und dem Server oder dem Client.

Aus der DE 40 23 785 A1 ist ein System zur Speicherung, Bereitstellung und Aktualisierung von festen und/oder variablen Patienten- und Behandlungsdaten beschrieben. Das System umfasst eine stationäre Computer-Zentraleinheit und eine mobile Einheit. Die mobile Einheit wird bei Hausbesuchen und Visiten unter Zuhilfenahme einer Krankenversicherungskarte eingesetzt.

Die Aufgabe der Erfindung ist daher, ein Verfahren und eine Vorrichtung anzugeben, das bzw. die Voraussetzungen für eine leichte Überprüfung von Kodierungen im Gesundheitswesen schafft.

Die erste Aufgabe wird gelöst durch ein Verfahren für eine Überprüfung von einem Patienten zugeordneten Kodierungen im Gesundheitswesen, aufweisend folgende Verfahrensschritte:
- Empfangen von einem Patienten zugeordneten Kodierungen im Gesundheitswesen mit einer zentralen Datenbank, wobei die Kodierungen einem medizinischen Profil des Patienten zugeordnet sind,
- Empfangen von zur Beschreibung der Behandlung des Patienten geeigneten medizinischen Daten mit der Datenbank und
- Überprüfen, ob die Kodierungen der tatsächlichen Behandlung des Patienten zugeordnet werden können.

Erfindungsgemäß werden also die dem Patienten zugeordneten und sein medizinisches Profil beschreibenden Kodierungen an die zentrale Datenbank übermittelt. Diese Kodierungen können beispielsweise, wie es nach einer Variante der Erfindung vorgesehen ist, auf dem International Classification of Diseases (ICD), dem International Classification of Prozedures in Medizin (ICPM) und/oder dem Diagnosis Related Groups (DRG) Kodierungssystem basieren. Umfasst das medizinische Profil eine Art der Erkrankung des Patienten, könnte somit beispielsweise das ICD- oder das DRG-Kodierungssystem verwendet werden. Eine an dem Patienten durchgeführte medizinische Prozedur könnte nach dem ICPM-Kodierungssystem kodiert werden. Unter Prozeduren werden in diesem Zusammenhang beispielsweise Untersuchungen insbesondere mit medizintechnischen Geräten oder operative Eingriffe am Patienten verstanden.

Ferner werden erfindungsgemäß zur Beschreibung der Behandlung des Patienten geeignete medizinische Daten an die Datenbank übertragen. Diese Daten können ebenfalls kodiert sein und können u.a. gemäß einer Variante der Erfindung eine Angabe über verschriebene Medikamente, durchgeführte Untersuchungen und/oder durchgeführte Labortests des Patienten umfassen.

Aufgrund der medizinischen Daten wird erfindungsgemäß überprüft, ob die Behandlung des Patienten mit den Kodierungen übereinstimmt. Eine Überprüfung der Kodierung aufgrund eines Medikamentes ist z.B. eine Überprüfung, ob ein Patient, bei dem ein behandelnder Arzt eine Diagnose Hypertonie erstellte und entsprechend kodierte, auch ein antihypertensives Medikament bekam. Ein weiteres Beispiel ist eine Diagnose Hepatitis für einen Patienten, die auch entsprechend kodiert wurde. Bei einer diagnostizierten Hepatitis müsste eine Hepatitisserologie, also ein geeigneter Labortest, vorgenommen worden sein. Also müssen bei einer korrekten Kodierung der Diagnose Hepatitis auch medizinische Daten, die einer Hepatitisserologie zugeordnet werden können, vorliegen. Außerdem müsste die vorgenommene Hepatitisserologie hinweisend auf eine Hepatitis sein.

Aufgrund der Behandlung des Patienten kann also ein Rückschluss auf die dem Patienten zugeordneten Kodierungen geschlossen und somit die Kodierungen überprüft werden.

Gemäß einer Variante der Erfindung sind die medizinischen Daten wenigstens einem schriftlichen Dokument, das insbesondere ein Arztbrief ist, zugeordnet. Bei einer Behandlung des Patienten z.B. in einem Krankenhaus wird normalerweise bei der Entlassung des Patienten aus dem Krankenhaus dem Hausarzt des Patienten, also dem Arzt, den der Patient regelmäßig aufsucht, ein Arztbrief zugestellt. Der Arztbrief umfasst in der Regel eine Angabe über die Diagnose des Patienten, eine durchgeführte Behandlung, verordnete Medikamente und/oder eine Empfehlung für eine weitere Behandlung des Patienten nach dem Krankenhausaufenthalt. Diese Angaben können wiederum einen Rückschluss auf die dem Patienten zugeordneten Kodierungen erlauben; diese Angaben erlauben also auch eine Überprüfung der dem Patienten zugeordneten Kodierungen.

Die dem schriftlichen Dokument zugeordneten Daten können nach einer besonders bevorzugten Variante der Erfindung mittels Mittel zur Textanalyse nach speziellen Schlagwörtern ausgewertet werden, wobei die Schlagwörter beispielsweise einen Rückschluss auf die Behandlung des Patienten zulassen. Die Schlagwörter sind z.B. Krankheiten, Medikamente, Diagnosen oder am Patienten durchgeführte medizinische Prozeduren. Somit kann die Auswertung des schriftlichen Dokuments und somit die Überprüfung der Kodierungen in einfacher Weise automatisiert durchgeführt werden.

Um eine möglichst breit anwendbare Übertragung der medizinischen Daten zu gewährleisten, werden diese gemäß einer besonders bevorzugten Variante der Erfindung mittels des Health Level 7 (HL7), des Befunddatenträgers (BDT), des VCS Formats, des Digital Imaging and Communications in Medicine (DICOM) Standards und/oder des Labordatenträger (LDT) Formats übermittelt. Bei diesen Formaten handelt es sich um spezielle Kodierverfahren, die vor allem in Krankenhäusern zur Übermittlung von Labordaten verwendet werden.

Nach einer weiteren Ausführungsform der Erfindung sind den medizinischen Daten wenigstens ein medizinisches Bild des Patienten zugeordnet, das mittels eines bildgebenden medizinischen Gerätes hergestellt wurde. Das medizintechnische Gerät ist gemäß einer Ausführungsform der Erfindung ein Röntgengerät, ein Computertomograph, ein Magnetresonanzgerät und/oder ein Ultraschallgerät. Das medizinische Bild kann gemäß einer Ausführungsform der Erfindung mittels Mittel zur Bildverarbeitung ausgewertet und die Auswertung des medizinischen Bildes zur Überprüfung, ob die tatsächliche Behandlung des Patienten den Kodierungen zugeordnet werden kann, verwendet werden. Handelt es sich bei dem medizinischen Bild beispielsweise um ein Röntgenbild eines Patienten, der sich eine Knochenfraktur zugezogen hat, so können die Mittel zur Bildverarbeitung derart ausgeführt werden, dass sie aufgrund der dem Röntgenbild zugeordneten Bilddaten die Knochenfraktur automatisch erkennen. Wenn nun das Röntgenbild eine Knochenfraktur zeigt, erscheint eine dem Patienten zugeordnete Kodierung, die der Diagnose Knochenfraktur des Patienten entspricht, plausibel.

Gemäß einer besonders bevorzugten Ausführungsform der Erfindung wird mit einer der Datenbank zugeordneten und ein wissensbasiertes System umfassenden Auswerteeinrichtung überprüft, ob die tatsächliche Behandlung des Patienten den Kodierungen zugeordnet werden kann. Das wissensbasierte System umfasst dabei gemäß einer Variante der Erfindung ein neuronales Netz, ein kausal probalistisches Netz, ein Fuzzy-System, ein regelbasiertes System und/oder einen Entscheidungsbaum.

Das erfindungsgemäße Verfahren ist besonders interessant, wenn gemäß einer Variante der Erfindung die Datenbank von einem Krankenhaus, einer Krankenkasse, einer Krankenversicherung und/oder einem ärztlichen Abrechnungszentrum betrieben wird. Wird die Datenbank von dem Krankenhaus betrieben, kann es mit einem Krankenhausinformationssystem verbunden sein, wodurch in einfacher Weise die dem Patienten zugeordneten Kodierungen und die zur Beschreibung der tatsächlichen Behandlung des Patienten geeigneten medizinischen Daten an die Datenbank übermittelt werden können.

Die zweite Aufgabe der Erfindung wird gelöst durch eine Vorrichtung für eine Überprüfung von einem Patienten zugeordneten Kodierungen im Gesundheitswesen, aufweisend
- eine zentrale Datenbank, an die den Kodierungen zugeordnete Daten und zur Beschreibung der Behandlung des Patienten geeignete medizinische Daten übermittelbar sind, wobei die Kodierungen einem medizinischen Profil des Patienten zugeordnet sind, und
- eine Auswerteeinrichtung mit einem wissensbasierten System, das die der Kodierung zugeordneten Daten und die der Behandlung des Patienten zugeordneten Daten auf Plausibilität prüft .

Die erfindungsgemäße Vorrichtung kann also zum Durchführen des erfindungsgemäßen Verfahrens verwendet werden.

Vorteilhafte Ausgestaltungen der erfinderischen Vorrichtung ergeben sich aus den Unteransprüchen.

Ausführungsbeispiele sind in den beigelegten schematischen Zeichnungen exemplarisch dargestellt. Es zeigen:
Fig. 1 eine Arztpraxis eines Kardiologen,
Fig. 2 ein das erfindungsgemäße Verfahren veranschaulichendes Flussdiagramm und
Fig. 3 ein Krankenhaus.

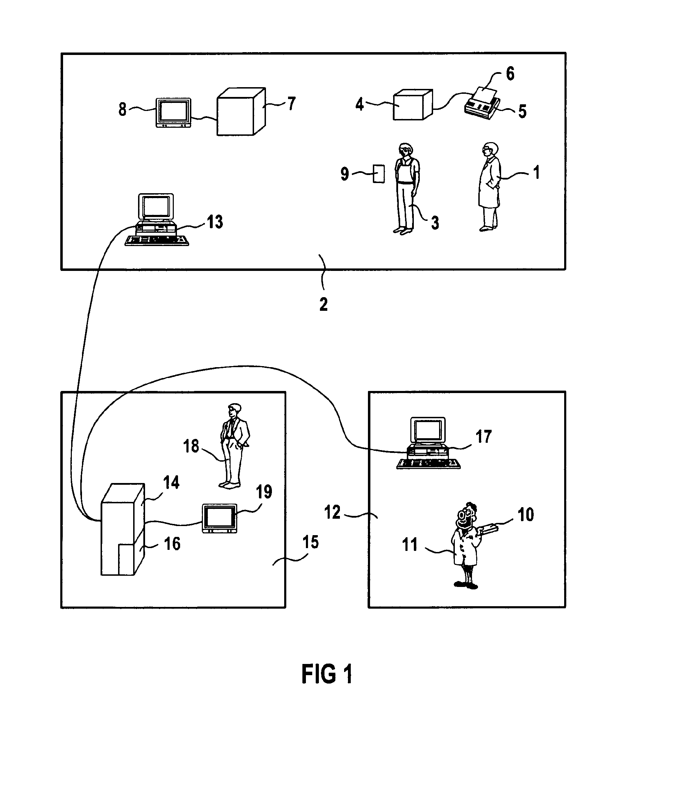

Die Fig. 1 zeigt einen Kardiologen 1 in seiner Arztpraxis 2, der einen Patienten 3 untersucht. Im Falle des vorliegenden Ausführungsbeispiels klagt der Patient 3 über Herzbeschwerden, weshalb der Kardiologe 1 ein EKG von dem Patienten 3 mit einem in der Arztpraxis 2 befindlichen EKG-Gerät 4 erstellt. Während der Erstellung des EKGs erstellt ein mit dem EKG-Gerät 4 verbundener Drucker 5 einen Ausdruck 6 des EKGs. Ferner untersucht der Kardiologe 1 das Herz des Patienten 3 mit einem in der Arztpraxis 2 befindlichen Ultraschallgerät 7. Die während der Untersuchung des Patienten 3 mit dem Ultraschallgerät 7 hergestellten Bilder des Herzens des Patienten 3 kann der Kardiologe 1 auf einem mit dem Ultraschallgerät 7 verbundenen Monitor 8 betrachten.

Aufgrund des Ausdrucks 6 des EKGs und der Untersuchung mit dem Ultraschallgerät 7 diagnostiziert der Kardiologe 1 eine Herzinsuffizienz des Patienten 3. Im Falle des vorliegenden Ausführungsbeispieles ist die Herzinsuffizienz relativ leicht ausgeprägt, so dass der Kardiologe 1 dem Patienten 3 ein Diuretikum 10 als Medikament verschreibt und ihm ein entsprechendes Rezept 9 übergibt.

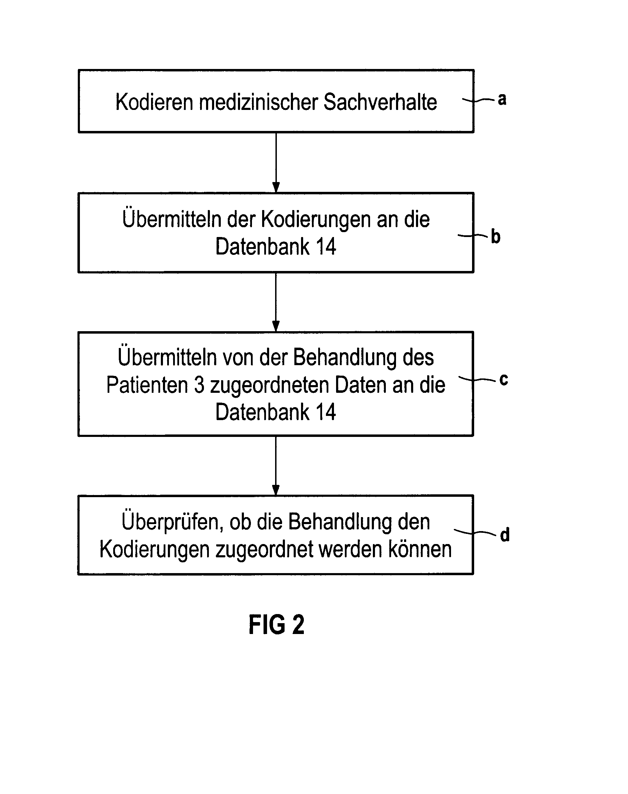

Nachdem der Patient 3 die Arztpraxis 2 des Kardiologen 1 verlassen hat, kodiert der Kardiologe 1 die Herzinsuffizienz des Patienten 3 entsprechend dem in Deutschland gebräuchlichen ICD (International Classification of Diseases) Kodierungssystem. Die entsprechende ICD Kodierung lautet I11.0. Ferner kodiert der Kardiologe 1 die am Patienten 3 durchgeführten Untersuchungen, also das erstellte EKG und die Untersuchung mit dem Ultraschallgerät 7 entsprechend dem in Deutschland gebräuchlichen ICPM (International Classification of Prozedures in Medicine) Kodierungssystem. Die Kodierungen für das erstellte EKG und für die Untersuchung mit dem Ultraschallgerät 7 lauten 1-724 bzw. 5-952.0 (Schritt a des in der Fig. 2 dargestellten Flussdiagramms).

Anschließend speichert der Kardiologe 1 die ermittelten Kodierungen in einem in der Arztpraxis 2 befindlichen Rechner 13 ab, in der eine elektronische Krankenakte des Patienten 3 gespeichert ist. Danach übermittelt der Kardiologe 1 die Kodierungen mit der Identität des Patienten 3 an eine Datenbank 14, die sich in einem Geschäftsraum 15 der Krankenkasse des Patienten 3 befindet und die übermittelten Kodierungen empfängt. Die Kodierungen sind im Übrigen Grundlage einer Bezahlung des Kardiologen 1 von der Krankenkasse des Patienten 3. Im Falle des vorliegenden Ausführungsbeispieles werden die Kodierungen über das Internet übermittelt, an das der Rechner 13 und die Datenbank 14 angeschlossen sind (Schritt b des in der Fig. 2 dargestellten Flussdiagramms).

Nachdem der Patient 3 die Arztpraxis 2 des Kardiologen 1 verlassen hat, geht er zu einer Apotheke 12, die einem Apotheker 11 gehört, der dem Patienten 3 das von dem Kardiologen 1 verschriebene Diuretikum 10 aufgrund des ihm von dem Patienten 3 ausgehändigten Rezeptes 9 übergibt. Somit kann der Patient 3 eine Behandlung seiner Herzinsuffizienz beginnen, indem er das von dem Kardiologen 1 verschriene Diuretikum 10 gemäß einer Anweisung des Kardiologen 1 einnimmt.

Anschließend gibt der Apotheker 11 die Abgabe des Diuretikums 10 an den Patienten 3 in einem in der Apotheke 12 befindlichen und an das Internet angeschlossenen Rechner 17 ein. Die Information der Abgabe des Diuretikums 10 an den Patienten 3 wird ebenfalls an die Datenbank 14 der Krankenkasse des Patienten 3 übermittelt, so dass der Apotheker 11 in geeigneter Weise von der Krankenkasse des Patienten 3 für die Abgabe des Diuretikums 10 vergütet werden kann (Schritt c des in der Fig. 2 dargestellten Flussdiagramms).

Im Falle des vorliegenden Ausführungsbeispieles empfängt also die Datenbank 14 dem Patienten 3 zugeordnete Kodierungen und Daten, die der Behandlung des Patienten 3 zugeordnet sind. Die Kodierungen sind der Diagnose für den Patienten 3 und den am Patienten 3 durchgeführten medizinischen Prozeduren, also einem medizinischen Profil des Patienten 3, zugeordnet. Die Behandlung des Patienten 3 besteht in der Einnahme des vom Kardiologen 1 verschriebenen Diuretikums 10.

Im Falle des vorliegenden Ausführungsbeispiels überprüft eine der Datenbank 14 zugeordnete Auswerteeinrichtung 16 mit einem geeigneten Rechnerprogramm die an die Datenbank 14 von dem Kardiologen 1 übermittelten Kodierungen, indem es in der Datenbank 14 gespeicherte Zuordnungen von Kodierungen mit zu erwartenden Behandlungen mit den von dem Kardiologen 1 übermittelten Kodierungen und der Behandlung des Patienten 3 vergleicht. (Schritt d des in der Fig. 2 dargestellten Flussdiagramms).

Im Falle des vorliegenden Ausführungsbeispieles diagnostizierte der Kardiologe 1 beim Patienten 3 eine Herzinsuffizienz. Bei einer Herzinsuffizienz ist es im Falle des vorliegenden Ausführungsbeispiels dem Rechnerprogramm vorgegeben, dass ein Arzt, um diese Diagnose zu erstellen, zumindest ein EKG erstellt, aber auch eine Ultraschalluntersuchung durchführen kann. Eine zu erwartende Behandlung einer Herzinsuffizienz ist die Einnahme eines Diuretikums oder ein Einsetzen eines Herzschrittmachers, je nach Ursache der Herzinsuffizienz.

Im Falle des vorliegenden Ausführungsbeispieles überprüft das Rechnerprogramm, ausgehend von der DRG-Kodierung "I11.0" ("Herzinsuffizienz"), ob ein EKG erstellt und eventuell eine Ultraschalluntersuchung am Patienten 3 durchgeführt wurde. Anschließend überprüft das Rechnerprogramm, ob die erfolgte Behandlung des Patienten 3 der Diagnose "Herzinsuffizienz" zugeordnet werden kann.

Im Falle des vorliegenden Ausführungsbeispiels erstellte der Kardiologe 1 sowohl ein EKG und führte eine Ultraschalluntersuchung durch. Aufgrund der entsprechenden Kodierungen, die der Kardiologe 1 der Datenbank 14 übermittelte, ist dies dem in der Auswerteeinrichtung 16 gespeicherten Rechnerprogramm bekannt. Außerdem verschrieb der Kardiologe 1 dem Patienten 3 das Diuretikum 10, welches dem Patienten 3 vom Apotheker 11 ausgehändigt wurde. Dies ist dem Rechnerprogramm ebenfalls bekannt, so dass es aufgrund der an die Datenbank 14 übertragenen Daten erkennt, dass die Kodierungen des Kardiologen 1 plausibel sind.

Sollte das Rechnerprogramm eine unplausible Kodierung ermitteln, weil sich der Kardiologe 1 beispielsweise bei der Eingabe der Kodierungen irrte und z.B. anstelle einer Ultraschalluntersuchung eine Behandlung mit einem Lithotripter kodierte, wird dies von dem Rechnerprogramm erkannt. Daraufhin erscheint auf einem mit der Datenbank 14 verbundenen Monitor 19 ein Hinweis, der Kardiologe 1 habe eine unplausible Kodierung an die Datenbank 14 übermittelt. Dieser Hinweis ist für einen Angestellten 18 der Krankenkasse des Patienten 3 bestimmt, der daraufhin die unplausible Kodierung überprüfen und gegebenenfalls den Kardiologen 1 kontaktieren kann.

Sollte außerdem der Ausdruck 6 des von dem Kardiologen 1 durchgeführten EKGs an den Patienten 3 in digitaler Form vorliegen, könnten die dem Ausdruck 6 zugeordneten Daten ebenfalls an die Datenbank 14 übermittelt werden. Dann kann das in der Auswerteeinrichtung 16 gespeicherte Rechnerprogramm mittels eines Mustererkennungsalgorithmus diese Daten auf einen Hinweis einer Herzinsuffizienz analysieren. Sollte es einen Hinweis auf eine Herzinsuffizienz geben, ist die kodierte Diagnose "Herzinsuffizienz" plausibel, ansonsten ist sie unplausibel.

Auch ein von dem Kardiologen 1 erstelltes Dokument, beispielsweise ein Arztbrief, könnte in digitalisierter Form an die Datenbank 14 übermittelt werden. Dieses Dokument könnte dann mit einem geeigneten Textanalyse-Algorithmus mit dem in der Auswerteeinrichtung 16 gespeicherten Rechnerprogramm z.B. auf bestimmte Schlagwörter durchsucht werden, die einen Rückschluss auf die gestellte Diagnose und somit auf die Kodierungen zulassen.

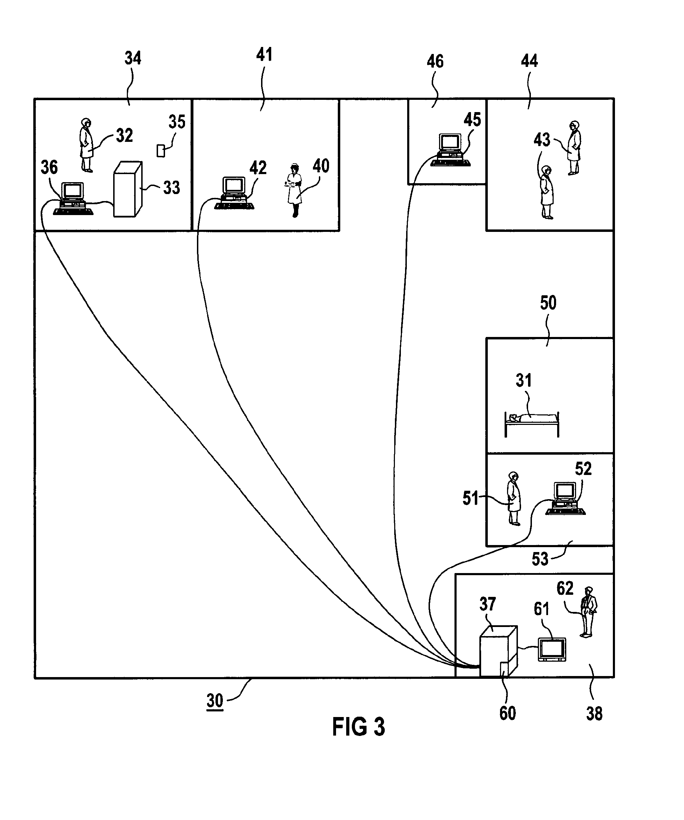

Ein weiteres Ausführungsbeispiel zur Veranschaulichung des erfindungsgemäßen Verfahrens und des erfindungsgemäßen Systems ist in der Fig. 3 schematisch dargestellt.

Die Fig. 3 zeigt ein Krankenhaus 30, in welches ein Patient 31 von einem in der Fig. 3 nicht dargestellten Rettungsteam eingeliefert wurde. Der Patient 31 hatte im Falle des vorliegenden Ausführungsbeispieles einen Verkehrsunfall und klagt über einen schmerzhaften Oberschenkel.

Nach der Einlieferung in das Krankenhaus 30 wird dem Patienten 31 Blut abgenommen, das von einer Laborantin 40 in einem Labor 41 des Krankenhauses 30 untersucht wird. Die Laborantin 40 ermittelt außerdem eine der Laboruntersuchung zugeordnete ICPM-Kodierung, die sie in einen Rechner 42 eingibt und an eine Datenbank 37 übermittelt. Der Rechner 42 befindet sich in dem Labor 41 und die Datenbank 37 befindet sich in einem Verwaltungsraum 38 des Krankenhauses 30. Der Rechner 42 ist außerdem mit der Datenbank 37 verbunden.

Anschließend erstellt ein Radiologe 32 mit einem Röntgengerät 33, das sich in einer radiologischen Abteilung 34 des Krankenhauses 30 befindet, ein Röntgenbild 35 des schmerzenden Oberschenkels des Patienten 31. Aufgrund des Röntgenbildes 35 diagnostiziert der Radiologe 32 eine komplizierte Oberschenkelfraktur des Patienten 31. Danach ermittelt der Radiologe 32 eine für die Röntgenuntersuchung zugeordnete ICPM-Kodierung und eine der Diagnose "Oberschenkelfraktur" zugeordnete ICD-Kodierung und gibt beide Kodierungen in einen Rechner 36 ein, welcher sich ebenfalls in der radiologischen Abteilung 34 befindet. Anschließend übermittelt er beide Kodierungen an die Datenbank 37, mit der der Rechner 36 verbunden ist. Im Falle des vorliegenden Ausführungsbeispieles ist das Röntgengerät 33 außerdem mit dem Rechner 36 verbunden, so dass ein dem Röntgenbild 35 zugeordneter Bilddatensatz mit dem Rechner 36 ebenfalls an die Datenbank 37 übermittelt wird.

Aufgrund des Röntgenbildes 35 wird der Patient 31 ferner von einem Operationsteam 43 in einem Operationssaal 44 des Krankenhauses 30 operiert. Nach der Operation gibt einer der Ärzte des Operationsteams 43 eine der Operation zugeordnete ICPM-Kodierung in einen Rechner 45 ein, der sich in einem Nebenraum 46 des Operationssaales 44 befindet. Der Rechner 45 ist ebenfalls mit der Datenbank 37 verbunden, so dass die der Operation zugeordnete ICPM-Kodierung ebenfalls an die Datenbank 37 übermittelt wird.

Nach der Operation wird der Patient 31 auf eine Station 50 des Krankenhauses 30 verlegt, auf der er im Falle des vorliegenden Ausführungsbeispieles zehn Tage stationär behandelt wird. Ein Stationsarzt 51 gibt die dieser Behandlung zugeordnete ICPM-Kodierung in einen Rechner 52 ein, der sich in einem Raum 53 der Station 50 befindet und ebenfalls mit der Datenbank 37 verbunden ist, so dass die von dem Stationsarzt 51 eingegebene ICPM-Kodierung ebenfalls an die Datenbank 37 übertragen wird.

Im Falle des vorliegenden Ausführungsbeispieles umfasst die Datenbank 37 eine Auswerteeinrichtung 60, in welcher ein Rechnerprogramm gespeichert ist, das die dem Patienten 31 zugeordneten und an die Datenbank 37 übermittelten Kodierungen auf Plausibilität überprüft. Das Rechnerprogramm überprüft dabei, ob die beim Patienten 31 durchgeführte Behandlung, also die Operation und der stationäre Aufenthalt, die durchgeführten medizinerischen Prozeduren, also die Laboruntersuchung, das Röntgen des Oberschenkels und die Operation, aufgrund der erstellten Diagnose "Oberschenkelfraktur" plausibel sind, indem es erst die dem Patienten 31 zugeordneten Kodierungen interpretiert und mit in der Datenbank 37 gespeicherten Zuordnungen von Behandlungen und Diagnosen vergleicht. Im Falle des vorliegenden Ausführungsbeispieles ist in der Datenbank 37 gespeichert, dass bei einer Diagnose "Oberschenkelfraktur" ein Röntgenbild des Oberschenkels zu erwarten ist. Handelt es sich um eine komplizierte Oberschenkelfraktur, kann auch der Diagnose "Oberschenkelfraktur" eine Operation mit nachfolgendem stationären Aufenthalt zugeordnet sein. Eine komplizierte Oberschenkelfraktur kann aus dem Röntgenbild 35 des Oberschenkels abgeleitet werden. Daher umfasst die Auswerteeinrichtung 60 ein geeignetes Mustererkennungsprogramm, mit dem ein einem Röntgenbild zugeordneter Bilddatensatz auf einen Hinweis auf eine Knochenfraktur analysiert werden kann. Das Mustererkennungsprogramm, das im Falle des vorliegenden Ausführungsbeispiels ein geeignet trainiertes neuronales Netz umfasst, ist ferner derart ausgeführt, dass es auch eine komplizierte von einer weniger komplizierten Knochenfraktur unterscheiden kann.

Im Falle des vorliegenden Ausführungsbeispiels analysiert das Mustererkennungsprogramm den an die Datenbank 37 übermittelten und dem Röntgenbild 35 zugeordneten Bilddatensatz und ermittelt, dass es sich um eine komplizierte Knochenfraktur handelt. Daher erkennt das in der Auswerteeinrichtung 60 gespeicherte Rechnerprogramm, dass die Diagnose "Oberschenkel-fraktur" richtig gestellt und somit die der Oberschenkelfraktur zugeordnete ICD-Kodierung richtig ist. Außerdem war die Operation und der stationäre Aufenthalt des Patienten 31 aufgrund des Röntgenbildes 35 plausibel, so dass auch die der Operation und dem stationären Kranhausaufenthalt zugeordneten ICPM-Kodierungen plausibel sind. Eine Laboruntersuchung wird im Übrigen von jedem Patienten routinemäßig durchgeführt, so dass die entsprechende ICPM-Kodierung des Patienten 31 ebenfalls als plausibel erkannt wird.

Sollte das Rechnerprogramm eine unplausible Kodierung ermitteln, weil z.B. ein medizinischer Sachverhalt falsch kodiert wurde, wird dies auf einem Anzeigegerät 61, welches mit der Datenbank 37 verbunden ist, angezeigt, so dass ein Angestellter 62 des Krankenhauses 30 über die unplausible Kodierung informiert wird und die Kodierungen entsprechend überprüfen kann.

Das Mustererkennungsprogramm muss übrigens nicht notwendigerweise ein neuronales Netz umfassen. Es kann insbesondere auch ein kausal-probalistisches Netz, ein Fuzzy System, ein regelbasiertes System und/oder einen Entscheidungsbaum umfassen.

Andere oder weitere Kodiersysteme können auch verwendet werden, wie insbesondere das DRG-kodierungssystem.

## Patentansprüche

1. Verfahren für eine Überprüfung von einem Patienten zugeordneten Kodierungen im Gesundheitswesen, aufweisend folgende Verfahrensschritte:
- Empfangen von einem Patienten (3, 31) zugeordneten Kodierungen im Gesundheitswesen mit einer zentralen Datenbank (14, 37), wobei die Kodierungen einem medizinischen Profil des Patienten (3, 31) zugeordnet sind,
- Empfangen von zur Beschreibung der Behandlung des Patienten (3, 31) geeigneten medizinischen Daten mit der Datenbank (14, 37) und
- Überprüfen, ob die Kodierungen der tatsächlichen Behandlung des Patienten (3, 31) zugeordnet werden können.

2. Verfahren nach Anspruch 1, bei dem die Kodierungen auf dem International Classification of Diseases (ICD), dem International Classification of Procedures in Medicine (ICPM) und/oder den Diagnosis Related Groups (DRG) Kodierungssystem basieren.

3. Verfahren nach Anspruch 1 oder 2, bei dem die medizinischen Daten eine Angabe über verschriebene Medikamente (10), durchgeführter Untersuchungen und/oder durchgeführter Labortests des Patienten (3, 31) umfassen.

4. Verfahren nach einem der Ansprüche 1 bis 3, bei dem die medizinischen Daten wenigstens einem schriftlichen Dokument, das insbesondere ein Arztbrief ist, zugeordnet sind.

5. Verfahren nach Anspruch 4, bei dem die dem schriftlichen Dokument zugeordneten Daten mittels Mittel zur Textanalyse nach speziellen Schlagwörtern ausgewertet werden, wobei die Schlagwörter einen Rückschluss auf die tatsächliche Behandlung des Patienten (3, 31) zulassen.

6. Verfahren nach einem der Ansprüche 1 bis 5, bei dem die medizinischen Daten mittels des Health Level 7 (HL7), des Befunddatenträgers (BDT), des VCS Formats, des Digital Imaging and Communications in Medicine (DICOM) Standards und/oder des Labordatenträger (LDT) Formats übermittelt werden.

7. Verfahren nach einem der Ansprüche 1 bis 6, bei dem die medizinischen Daten wenigstens einem medizinischen Bild (35) des Patienten (31) zugeordnet sind, das mittels eines bildgebenden medizintechnischen Gerätes (33) hergestellt wurde.

8. Verfahren nach Anspruch 7, bei dem das bildgebende medizintechnische Gerät ein Röntgengerät (33), ein Computertomograf, ein Magnetresonanzgerät und/oder ein Ultraschallgerät (7) ist.

9. Verfahren nach Anspruch 7 oder 8, bei dem das medizinische Bild (35) mittels Mittel zur Bildverarbeitung ausgewertet wird und die Auswertung des medizinischen Bildes (35) zur Überprüfung, ob die tatsächliche Behandlung des Patienten (31) den Kodierungen zugeordneten werden kann, verwendet wird.

10. Verfahren nach einem der Ansprüche 1 bis 9, bei dem mittels einer der Datenbank (14, 37) zugeordneten und ein wissensbasiertes System umfassenden Auswerteeinrichtung (16, 60) überprüft wird, ob die Behandlung des Patienten (3, 31) den Kodierungen zugeordnet werden kann.

11. Verfahren nach Anspruch 10, bei dem das wissensbasierte System ein neuronales Netz, ein kausal-probalistisches Netz, ein Fuzzy System, ein regelbasiertes System und/oder einen Entscheidungsbaum umfasst.

12. Verfahren nach einem der Ansprüche 1 bis 11, bei dem die Datenbank (14, 37) von einem Krankenhaus (30), einer Krankenkasse, einer Krankenversicherung und/oder einem ärztlichen Abrechnungszentrum betrieben wird.

13. Vorrichtung für eine Überprüfung von einem Patienten zugeordneten Kodierungen im Gesundheitswesen, aufweisend
- eine zentrale Datenbank (14, 37), an die den Kodierungen zugeordnete Daten und zur Beschreibung der Behandlung des Patienten (3, 31) geeignete medizinische Daten übermittelbar sind, wobei die Kodierungen einem medizinischen Profil des Patienten (3, 31) zugeordnet sind, und
- eine Auswerteeinrichtung (16, 60) mit einem wissensbasierten System, das die der Kodierung zugeordneten Daten und die der Behandlung des Patienten (3, 31) zugeordneten Daten auf Plausibilität prüft.

14. Vorrichtung nach Anspruch 13, bei der die Kodierungen auf dem International Classification of Diseases (ICD), dem International Classification of Procedures in Medicine (ICPM) und/oder den Diagnosis Related Groups (DRG) Kodierungssystem basieren.

15. Vorrichtung nach Anspruch 13 oder 14, bei der die medizinischen Daten eine Angabe über verschriebene Medikamente (10), durchgeführter Untersuchungen und/oder durchgeführter Labortests des Patienten (3, 31) umfassen.

16. Vorrichtung nach einem der Ansprüche 13 bis 15, bei der die medizinischen Daten wenigstens einem schriftlichen Dokument, das insbesondere ein Arztbrief ist, zugeordnet sind.

17. Vorrichtung nach Anspruch 16, bei der die dem schriftlichen Dokument zugeordneten Daten mittels Mittel zur Textanalyse nach speziellen Schlagwörtern auswertbar sind, wobei die Schlagwörter einen Rückschluss auf die tatsächliche Behandlung des Patienten (3, 31) zulassen.

18. Vorrichtung nach einem der Ansprüche 13 bis 17, bei der die medizinischen Daten mittels mittels des Health Level 7 (HL7), des Befunddatenträgers (BDT), des VCS Formats, des Digital Imaging and Communications in Medicine (DICOM) Standards und/oder des Labordatenträger (LDT) Formats übermittelbar sind.

19. Vorrichtung nach einem der Ansprüche 13 bis 18, bei der die medizinischen Daten wenigstens einem medizinischen Bild (35) des Patienten (31) zugeordnet sind, das mittels eines bildgebenden medizintechnischen Gerätes (33) hergestellt wurde.

20. Vorrichtung nach Anspruch 19, bei der das bildgebende medizintechnische Gerät ein Röntgengerät (33), ein Computertomograf, ein Magnetresonanzgerät und/oder ein Ultraschallgerät ist.

21. Vorrichtung nach Anspruch 19 oder 20, bei der das medizinische Bild (35) mittels Mittel zur Bildverarbeitung, die der Auswerteeinrichtung (60) zugeordnet sind, ausgewertet wird.

22. Vorrichtung nach Anspruch 21, bei der das wissensbasierte System ein neuronales Netz, ein kausal-probalistisches Netz, ein Fuzzy System, ein regelbasiertes System und/oder einen Entscheidungsbaum umfasst.

23. Vorrichtung nach einem der Ansprüche 13 bis 22, bei der die Datenbank (14, 37) von einem Krankenhaus (30), einer Krankenkasse, einer Krankenversicherung und/oder einem ärztlichen Abrechnungszentrum betrieben wird.
